# FASCICULE DE BREVET EUROPEEN

(11) **EP 3 374 522 B1**
(45) Date de publication et mention de la délivrance du brevet: **20.10.2021**
(21) Numéro de dépôt: 16805315.5
(22) Date de dépôt: 14.11.2016
(51) Int. Cl.: C12Q 1/68, G01N 27/327, G01N 33/543, B82Y 35/00

(54) **SYSTÈME DE DÉTECTION ÉLECTROCHIMIQUE DE MOLÉCULES D'INTÉRÊT**
ELEKTROCHEMISCHER SENSOR ZUM NACHWEIS VON ZIELMOLEKÜLEN
SYSTEM FOR ELECTROCHEMICAL DETECTION OF MOLECULES OF INTEREST

(30) Priorité: 13.11.2015 FR 1560917
(43) Date de publication de la demande: 19.09.2018
(73) Titulaire: Université Paris-Saclay, 91190 Gif-sur-Yvette (FR); Centre National de la Recherche Scientifique, 75016 Paris (FR)
(72) Inventeur: KORRI-YOUSSOUFI, Hafsa, 91680 Bruyeres le Chatel (FR); MIODEK, Anna, 91400 Orsay (FR); MEJRI, Nawel, 91220 Bretigny-sur-Orge (FR)
(74) Mandataire: IPAZ
(86) Numéro de dépôt international: PCT/EP2016/077564
(87) Numéro de publication internationale: WO 2017/081315

(56) Documents cités:
- WO-A1-2013/029115
- MIODEK A. ET AL.: "E-DNA sensor of Mycobacterium tuberculosis based on electrochemical assembly of nanomaterials (MWCNTs/PPy/PAMAM)", ANAL. CHEM., vol. 87, no. 18, 15 septembre 2015 (2015-09-15), pages 9257-9264, XP055264521,
- MIODEK A. ET AL.: "Electrochemical aptasensor of human cellular prion based on multiwalled carbon nanotubes modified with dendrimers: A platform for connecting redox markers and aptamers", ANAL. CHEM., vol. 85, no. 16, 20 août 2013 (2013-08-20) , pages 7704-7712, XP055264708,
- MIODEK A. ET AL.: "Electrochemical aptasensor of cellular prion protein based on modified polypyrrole with redox dendrimers", BIOSENS. BIOELECTRON., vol. 56, 15 juin 2014 (2014-06-15), pages 104-111, XP055265211,
- MIODEK A. ET AL.: "Direct electrochemical detection of PB1-F2 protein of influenza A virus in infected cells", BIOSENS. BIOELECTRON., vol. 59, septembre 2014 (2014-09), pages 6-13, XP55264701,
- LI F. ET AL.: "Carbon nanotube-polyamidoamine dendrimer hybrid-modified electrodes for highly sensitive electrochemical detection of microRNA24", ANAL. CHEM., vol. 87, no. 9, 5 mai 2015 (2015-05-05), pages 4806-4813, XP055264790,
- ZRIBI B. ET AL.: "A microfluidic electrochemical biosensor based on multiwall carbon nanotube/ferrocene for genomic DNA detection of Mycobacterium tuberculosis in clinical isolates", BIOMICROFLUIDICS, vol. 10, no. 1, 014115, 2 février 2016 (2016-02-02), pages 1-12, XP055264739,
- MIODEK A. ET AL.: "Electrochemical functionalization of polypyrrole through amine oxidation of poly(amidoamine) dendrimers: Application to DNA biosensor", TALANTA, vol. 154, 25 mars 2016 (2016-03-25), pages 446-454, XP55336649,

### SEQUENCE LISTING

<110> Université Paris-Sud
   Centre National de la Recherche Scientifique
<120> SYSTEME DE DETECTION ELECTROCHIMIQUE DE MOLECULES D'INTERET
<130> WOB-O15 UPS DAP
<160> 6
<170> PatentIn version 3.5
<210> 1
   <211> 15
   <212> DNA
   <213> Artificial Sequence
<220>
   <223> sonde ADNss
<400> 1
   gatacttcta tcacc 15
<210> 2
   <211> 15
   <212> DNA
   <213> Artificial Sequence
<220>
   <223> ADN d'intérêt synthétique
<400> 2
   ggtgatagaa gtatc 15
<210> 3
   <211> 15
   <212> DNA
   <213> Artificial Sequence
<220>
   <223> oligonucléotide non complémentaire
<400> 3
   cattccctc ttagg 15
<210> 4
   <211> 18
   <212> DNA
   <213> Artificial Sequence
<220>
   <223> sonde PCR
<400> 4
   ccgactgttg gcgctggg 18
<210> 5
   <211> 25
   <212> DNA
   <213> Artificial Sequence
<220>
   <223> sonde oligonucléotidique ciblant le virus de l'Hépatite C
<400> 5
   tcaacttcgg gaatctcaat gttag 25
<210> 6
   <211> 36
   <212> DNA
   <213> Artificial Sequence
<220>
   <223> sonde aptamère ciblant l'OTA
<400> 6
   gatcgggtgt gggtggcgta aagggagcat cggaca 36

## Description

La présente invention se rapporte à un système de détection électrochimique de molécules d'intérêt ainsi qu'à une méthode mettant en œuvre ledit système.

La mesure d'une molécule biologique, notamment d'un ADN ou d'une protéine pathogène, dans un échantillon est très utile et nécessaire dans différents domaines d'analyse tels que le diagnostic, l'environnement, ou l'alimentaire. Cette détection se fait actuellement par le biais de systèmes d'analyse nécessitant un environnement technique et des outils lourds, telle que la PCR.

Afin d'être utilisée dans le domaine d'analyse cliniques, le système de détection doit être sensible et permettre de quantifier dans un échantillon à analyser la présence des pathogènes même à de très faibles concentrations, par exemple de l'ordre de femtomolaire (10⁻¹⁵ mol/l), avec une très faible probabilité de faux-positifs.

Les dispositifs électrochimiques s'adaptent à cette problématique. Il est connu de l'art antérieur que les biosenseurs indiquent la présence d'une molécule d'intérêt, notamment un ADN, à l'aide d'une sonde ADN complémentaire à l'ADN d'intérêt et d'une sonde redox donnant un signal électrochimique.

Parmi les dispositifs de détection électrochimique de l'art antérieur, on distingue deux types de biosenseurs : les biosenseurs « signal-off » et les biosenseurs « signal-on ».

Les biosenseurs « signal-off » produisent une diminution de signal électrochimique après l'hybridation de la sonde ADN avec l'ADN d'intérêt. En effet, la formation d'un ADN double brin contraint la sonde redox à s'éloigner de la surface de l'électrode réduisant ainsi le transfert d'électrons entre la sonde redox et l'électrode.

Par exemple, Farjami et al. (Anal. Chem., 2011, 83 (5), pp 1594-1602) décrivent la détection d'ADN par une méthode électrochimique impliquant un biosenseur, lequel comporte:
- une sonde ADN en épingle à cheveux immobilisée par son extrémité 3' sur une électrode en or, et
- une sonde redox liée à l'extrémité 5' de ladite sonde ADN se trouvant à proximité de ladite électrode grâce à la structure en épingle à cheveux de la sonde ADN.

L'inconvénient de ce type de biosenseur est l'incertitude quant à l'origine de la diminution de signal qui peut être due à la dégradation ou au vieillissement de la sonde redox.

Les biosenseurs « signal-on » induisent une augmentation du signal électrochimique dès lors que la sonde ADN s'est hybridée avec l'ADN d'intérêt. Ces biosenseurs nécessitent :
- une structuration préalable particulière de la sonde ADN pour permettre d'éloigner la sonde redox de la surface d'électrode ou de la masquer, et
- une adaptation structurelle de la sonde ADN pour chaque ADN d'intérêt.

Lorsque le biosenseur entre en contact avec un ADN cible, l'interaction entre la sonde ADN et le biosenseur libère la sonde redox qui peut alors se rapprocher de l'électrode et créer un signal électrochimique.

La sonde ADN mise en œuvre dans ce type de biosenseur peut être sous forme d'une structure double brin, voire triple brin, et éventuellement configurée sous forme d'épingle à cheveux.

Xiao et al. (J. Am. Chem. Soc., 2007, 129, 11896-11897) décrivent un biosenseur électrochimique d'ADN comportant une sonde ADN simple brin marquée par une sonde redox. Ladite sonde ADN forme une structure en triple brin permettant d'éloigner la sonde redox de la surface de l'électrode. Lors de l'hybridation avec la sonde ADN, la sonde redox entre en contact avec la surface conductrice ayant pour conséquence une augmentation de courant. Le biosenseur électrochimique possède un seuil de sensibilité limité à 5nM.

Le principal inconvénient des biosenseurs de type « signal-on » connus est la structuration nécessaire et complexe de la sonde ADN qui exige une optimisation pour chaque ADN d'intérêt à détecter. Par ailleurs, ces biosenseurs électrochimiques d'ADN possèdent un seuil de sensibilité de détection non satisfaisant.

Ainsi, il existe toujours un besoin, notamment dans les domaines d'analyses cliniques et alimentaires, de développer un biosenseur électrochimique permettant la détection de molécules d'intérêt à de très faibles concentrations sans structuration préalable ou optimisation de la sonde ADN.

La présente invention a pour objet de mettre à disposition un système de détection électrochimique de type biosenseur « signal-on » dont la sensibilité est très élevée et dont l'adaptation de la sonde ADN n'est pas nécessaire.

Le système de détection électrochimique de molécules d'intérêt selon la présente invention comprend :
- un matériau conducteur,
- au moins un nanomatériau portant des fonctions positives, lié covalemment audit matériau conducteur,
- au moins une molécule redox, et
- au moins une sonde oligonucléotidique simple brin ciblant une molécule d'intérêt,
   ladite molécule redox étant lié covalemment audit nanomatériau ;
   ladite sonde oligonucléotidique étant liée covalemment audit nanomatériau ou à la molécule redox ;
le nombre de fonctions positives dudit nanomatériau étant supérieur au nombre de sondes oligonucléotidiques.

La présente invention repose sur le fait surprenant que l'interaction entre les charges négatives des groupements phosphates de la sonde oligonucléotidique et les charges positives du nanomatériau permet une organisation tridimensionnelle telle qu'elle bloque le transfert d'électrons de la molécule redox vers le matériau conducteur.

En l'absence de la molécule d'intérêt à détecter, les groupements phosphates chargés négativement interagissent avec les fonctions positives présentes à la surface du nanomatériau. Ces interactions de type ionique impliquent un repliement de la sonde oligonucléotidique sur le nanomatériau ayant pour effet d'empêcher le transfert d'électrons de la molécule redox vers la surface du nanomatériau.

Dès lors que la molécule d'intérêt à détecter est présente dans l'échantillon analysé, la réaction entre la molécule d'intérêt et la sonde oligonucléotidique change la conformation de la sonde oligonucléotidique, par exemple un ADN double brin se formant entre un ADN d'intérêt à détecter et la sonde oligonucléotidique, et interrompt l'interaction entre cette dernière et le nanomatériau, ce qui permet de démasquer la molécule redox et permettre le transfert d'électrons de la molécule redox vers la surface du nanomatériau.

Ce transfert d'électrons engendre un courant faradique qui peut être détecté par une méthode d'électroanalyse.

Dans le cadre de l'invention, l'intensité de ce courant est proportionnelle au nombre de molécules d'intérêt interagissant avec la sonde oligonucléotidique.

Par conséquent, la mesure du courant électrique permet d'indiquer la concentration de molécule d'intérêt dans l'échantillon analysé.

L'un des avantages du système de la présente invention est sa sensibilité élevée pour une molécule d'intérêt. Le système de la présente invention est capable de quantifier les molécules d'intérêt à de très faibles concentrations, notamment de l'ordre du femtomolaire (10⁻¹⁵ mol/L).

Un autre avantage du système de la présente invention est que la sonde oligonucléotidique simple brin ne nécessite ni structuration préalable particulière ni optimisation vis-à-vis des différentes molécules d'intérêt, du fait des interactions entre les charges négatives des groupements phosphates de la sonde oligonucléotidique et les charges positives du nanomatériau permettant une configuration tridimensionnelle telle que le transfert d'électrons est empêché.

De plus, contrairement aux systèmes de détection électrochimiques connus, le système de détection selon l'invention limite considérablement l'apparition de faux-positifs, grâce à la forte spécificité de la sonde oligonucléotidique et au fort blocage de transfert d'électrons.

Selon l'invention, on entend par « sonde oligonucléotidique » un oligonucléotide simple brin de 10 à 50 nucléotides, en particulier de 10 à 25 nucléotides, spécifique d'une molécule d'intérêt, dont la fonctionnalisation à l'extrémité 5' ou 3' permet la liaison covalente entre ladite sonde et la molécule redox.

A titre d'exemple, ledit oligonucléotide peut être fonctionnalisé par une amine ou un acide. L'amine ou l'acide peut être séparée de l'extrémité de l'oligonucléotide par un lieur hydrocarboné contenant 1 à 10 atomes de carbone.

Ledit oligonucléotide simple brin peut être un ARN ou un ADN, éventuellement formant un aptamère.

Par « molécule d'intérêt » susceptible d'être détectée par le système de détection électrochimique de l'invention, on peut citer un ADN, un ARN, une protéine, une toxine ou une molécule chimique.

Un ADN d'intérêt peut être un ADN génomique naturel isolé, un ADN viral, un ADNc synthétisé, ou un produit de PCR.

Un ARN d'intérêt peut être un ARNg, un ARNm, un pré-ARNm, un siARN, un microARN, un ARNi, un ARNt, un ARNr, un ARNsn, ou un ARN satellite.

Lorsque la molécule d'intérêt est un ADN ou un ARN, la sonde oligonucléotidique ciblant ladite molécule est un ADN ou un ARN ayant une séquence complémentaire. La présence de ladite molécule d'intérêt dans un échantillon conduit à la formation d'une structure double brin qui est placée perpendiculaire à la surface du nanomatériau.

Lorsque la molécule d'intérêt est une protéine ou une toxine, la sonde oligonucléotidique ciblant ladite molécule forme un aptamère sur lequel peut se fixer ladite molécule d'intérêt comme ligand. La séquence d'une telle sonde oligonucléotidique peut être déterminée selon les connaissances de l'Homme du métier ou sélectionnée selon une méthode conventionnelle, telle que la technique SELEX (évolution systématique de ligands par enrichissement exponentiel).

Conformément à la présente invention, on entend par « molécule redox » une molécule active pour une réaction d'oxydoréduction et capable de produire un signal électrochimique mesurable par une méthode d'électroanalyse liée au transfert d'électrons entre ladite molécule redox et le matériau conducteur. Ce signal électrochimique permet d'indiquer la présence d'une molécule d'intérêt complémentaire à la sonde oligonucléotidique.

Selon un mode de réalisation de l'invention, ladite molécule redox est liée de manière covalente à la fois audit nanomatériau et à la sonde oligonucléotidique, le nombre de fonctions positives dudit nanomatériau étant assez élevé afin de bloquer le transfert d'électrons de la molécule redox vers le matériau conducteur, et notamment le nombre de fonctions positives dudit nanomatériau étant supérieur au nombre de sondes oligonucléotidiques.

Ce type de système de détection selon l'invention permet une détection de plusieurs molécules d'intérêt simultanément.

Un mode de réalisation particulier de l'invention concerne un système de détection électrochimique comprenant :
- au moins deux types de molécules redox dont les potentiels d'oxydoréduction sont distincts, et
- au moins deux types de sondes oligonucléotidiques en simple brin chacun ciblant une molécule d'intérêt différente,
le même type de sondes oligonucléotidiques étant lié au même type de molécules redox.

L'utilisation d'au moins deux types de molécules redox chacun associé à un type de sonde oligonucléotidique dans ledit système de détection électrochimique permet de détecter simultanément au moins deux molécules d'intérêt différentes dans un échantillon. La présence de chaque molécule d'intérêt est indiquée respectivement par un signal électrochimique distinct.

Ledit système est particulièrement utile pour détecter les SNP (polymorphisme nucléotidique) dans un échantillon.

Dans un mode de réalisation avantageux, l'invention concerne un système de détection électrochimique comprenant :
- deux types de molécules redox dont les potentiels d'oxydoréduction sont distincts, et
- deux types de sondes oligonucléotidiques en simple brin, chacun ciblant une molécule d'intérêt différente,
le même type de sondes oligonucléotidiques étant lié au même type de molécules redox.

Dans un autre mode de réalisation avantageux, l'invention concerne un système de détection électrochimique comprenant :
- trois types de molécules redox dont les potentiels d'oxydoréduction sont distincts, et
- trois types de sondes oligonucléotidiques en simple brin, chacun ciblant une molécule d'intérêt différente,
le même type de sondes oligonucléotidiques étant lié au même type de molécules redox.

Selon un autre mode de réalisation de l'invention, le système de détection électrochimique de molécule(s) d'intérêt selon la présente invention comprend ladite molécule redox et ladite sonde oligonucléotidique liées covalemment uniquement audit nanomatériau, le nombre de fonctions positives dudit nanomatériau étant assez élevé afin de bloquer le transfert d'électrons de la molécule redox vers le matériau conducteur, et notamment le nombre de fonctions positives dudit nanomatériau étant supérieur au nombre de sondes oligonucléotidiques.

Ce type de système de détection présente l'avantage d'être moins fastidieux et plus rapide à synthétiser puisque la molécule redox présente un seul groupe fonctionnel.

La molécule redox utilisée dans le système de détection électrochimique de l'invention peut être toute molécule redox connue de l'Homme du métier, et plus particulièrement choisie dans le groupe comprenant le ferrocène, la quinone, le bleu de méthylène, les métalloporphyrines et le viologène.

Dans un mode de réalisation, ladite molécule redox peut être fonctionnalisée préalablement selon une méthode conventionnelle afin de la lier de manière covalente à un nanomatériau.

Par exemple, une molécule redox peut être fonctionnalisée par une acide pour introduire un groupe -COOH. Une molécule redox ainsi modifiée peut réagir, éventuellement à l'aide d'un agent de couplage, avec les amines situées en surface d'un nanomatériau. Plus particulièrement la molécule redox peut être fonctionnalisée de telle sorte à pouvoir réagir à la fois avec la sonde oligonucléotidique et au nanomatériau d'une manière covalente.

Dans un mode de réalisation où la sonde oligonucléotidique est liée covalemment au nanomatériau, ladite sonde est fonctionnalisée de manière à pouvoir réagir avec ledit nanomatériau sans réaction avec ladite molécule redox qui est liée également de manière covalente audit nanomatériau.

A titre d'exemple, une sonde oligonucléotidique peut être modifiée par un groupe -COOH afin de se lier covalemment avec les fonctions amines du nanomatériau.

Avantageusement, la fonctionnalisation préalable se fait par « chimie-click ».

Dans le cadre de la présente invention, les termes « molécule redox » et « sonde redox » peuvent être remplacés l'un par l'autre.

On entend par « nombre de fonctions positives dudit nanomatériau étant assez élevé » que le nombre de fonctions positives est suffisamment important pour permettre un repliement efficace des sondes oligonucléotidiques sur lesdites fonctions positives.

On entend par « le nombre de fonctions positives dudit nanomatériau étant supérieur au nombre de sondes oligonucléotidiques » que le nombre de fonctions positives portées par ledit nanomatériau est supérieur au nombre de sondes oligonucléotidiques liées de manière covalente au nanomatériau par l'intermédiaire d'une molécule redox, ce qui assure un blocage efficace du transfert d'électrons entre les charges négatives des groupements phosphates de la sonde oligonucléotidique et les charges positives du nanomatériau porteur de fonctions positives.

Le nombre de fonctions positives portées par un nanomatériau et le nombre de sondes oligonucléotidiques peuvent respectivement être déterminé selon les méthodes connues dans l'art antérieur.

Conformément à la présente invention, le nanomatériau portant des fonctions positives dudit système de détection électrochimique est choisi parmi le groupe comprenant :
- un dendrimère, préférentiellement un dendrimère de type de poly(amidoamine) notamment de deuxième, quatrième ou sixième génération (respectivement G2, G4 ou G6),
- des particules métalliques comme des nanoparticules d'or,
- des nanoparticules magnétiques, telles que l'oxyde de fer,
- des nanomatériaux hybrides à base de chitosan, et
- des polymères conducteurs éventuellement modifiés par des fonctions positives, tels que le polypyrrole, la polyaniline, ou le polyparaphénylène.

Dans un mode de réalisation avantageux, le nanomatériau utilisé selon le système de détection électrochimique de l'invention est un dendrimère de type poly(amidoamine) (PAMAM), dont la structure est globulaire et dont le diamètre ainsi que le nombre de groupes amines primaires présent à la surface dudit dendrimère augmentent en fonction du nombre de générations, aussi :
- les PAMAM G2 ont un diamètre d'environ 2,9 nm et possèdent 16 groupes amines primaires,
- les PAMAM G4 ont un diamètre d'environ 4,5 nm et possèdent 64 groupes amines primaires, et
- les PAMAM G6 ont un diamètre d'environ 6,7 nm et possèdent 256 groupes amines primaires.

L'utilisation des PAMAM portant une haute densité de groupes amines permet non seulement de faciliter la fonctionnalisation par différentes substances, telles que différentes molécules redox, mais aussi d'augmenter le seuil de la sensibilité dudit système de détection.

Dans un mode de réalisation plus avantageux, le nanomatériau utilisé dans un système de détection électrochimique de l'invention est les PAMAM G4.

Dans un autre mode de réalisation avantageux, le nanomatériau utilisé dans un système de détection de l'invention est des nanoparticules d'or fonctionnalisées. Les nanoparticules d'or ont un grand rapport surface sur volume permettant d'obtenir des matériaux structurés en trois dimensions utiles dans la construction de senseurs électrochimiques.

A titre d'exemple, les nanoparticules d'or peuvent être fonctionnalisées par la cystamine pour devenir un nanomatériau portant des fonctions positives. La cystamine comporte deux atomes de soufre pouvant s'attacher à la surface des nanoparticules d'or et laisser les groupements amines libres. Ces derniers peuvent être fonctionnalisés ultérieurement.

Dans un autre mode de réalisation avantageux, le nanomatériau utilisé dans un système de détection de l'invention est des nanoparticules d'or éventuellement fonctionnalisées associées aux PAMAM G4. Les nanoparticules d'or améliorent la conductivité et augmentent davantage le signal électrochimique. L'association des nanoparticules d'or avec des PAMAM permet d'élargir la gamme dynamique du système de détection de l'invention.

Le nanomatériau est lié au matériau conducteur par liaison covalente. L'Homme du métier saura choisir une méthode adaptée à la nature du nanomatériau pour le déposer sur le matériau conducteur.

Par exemple, les PAMAM peuvent être déposés à la surface d'une électrode en carbone par électrodéposition. L'oxydation des groupements amines des PAMAM créé un radical cationique -NH•⁺ qui interagit avec le groupement C=C en surface de carbone, mettant en œuvre ainsi un greffage covalent.

Les nanoparticules d'or fonctionnalisées par la cystamine peuvent être déposées, par l'intermédiaire des groupements amines, à la surface d'une électrode en carbone, en utilisant préalablement le N-(3-diméthylaminopropyl)-N'-éthylcarbodiimide hydrochloride/N-hydroxysuccinimide (EDC/NHS) pour fonctionnaliser ladite électrode.

Dans le système de détection électrochimique de l'invention, ledit matériau conducteur joue le rôle d'une électrode et fournit une surface régulière pour l'attache des nanomatériaux. Ledit matériau peut être formé de carbone, en particulier de nanotubes de carbone, de carbone vitreux, de graphite, de graphène ou d'un métal.

Dans un mode de réalisation particulier, ledit matériau conducteur est une micro- ou une nano-électrode qui peut être dispersée dans une solution.

Selon un autre mode de réalisation particulier de l'invention, le matériau conducteur est absent dudit système de détection lorsque ledit nanomatériau porteur de fonctions positives est conducteur.

Par exemple, des nanoparticules d'or, nanoparticules métalliques (platine, argent, ...), d'oxyde métalliques, de quantum dot, de graphite ou de graphène peuvent être à la fois le nanomatériau porteur de fonctions positives et le matériau conducteur.

Un système de détection électrochimique selon l'invention peut être utilisé dans :
- la détection des pathogènes, tels que les bactéries, les virus, les parasites, ou des biomarqueurs, tels que les biomarqueurs oncologiques, les biomarqueurs de la maladie d'Alzheimer, les biomarqueurs de la maladie de Parkinson..., dans un échantillon biologique, ce qui permet le diagnostic *in vitro* ou *in vivo* de maladies,
- l'identification de la résistance d'au moins un pathogène à un médicament,
- la détection de pathogène ou d'une toxine dans un échantillon agroalimentaire, pharmaceutique ou un effluent, par exemple les toxines bactériennes du genre *Salmonelle, Staphylocoque, Aeromona, Escherichia coli, Listéria, Clostridium,* ..., et
- le suivi de la présence ou l'absence d'au moins un médicament dans des fluides biologiques, notamment dans le cadre de traitements chroniques et/ou lourds comme la chimiothérapie ou la prise d'anticoagulant.

Pour ce faire, le système de détection électrochimique selon l'invention s'utilise selon les étapes suivantes :
i. mise en contact dudit système de détection électrochimique et de l'échantillon, et
ii. détection d'au moins un pathogène, d'au moins une toxine ou d'au moins un médicament d'intérêt.

A titre d'exemple, ledit système de détection électrochimique selon l'invention peut être utilisé dans la détection de l'Ochratoxine A.

Ledit système de détection électrochimique peut être aussi utilisé dans la détection ou dans la discrimination de sites SNP.

Selon un mode de réalisation particulier, ledit système de détection électrochimique de l'invention comporte une sonde oligonucléotidique de séquence SEQ ID NO : 4. Ledit système peut être utilisé pour détecter *Mycobacterium tuberculosis* résistant à la rifampicine en discriminant le site SNP (TCG/TTG) au sein du génome de *Mycobacterium tuberculosis.*

L'invention a également pour objet de proposer une méthode de détection électrochimique de molécules d'intérêt au sein d'un échantillon.

Ladite méthode comprend les étapes suivantes :
(i) la mise en contact d'un système de détection selon l'invention avec un échantillon susceptible de contenir une molécule d'intérêt, et
(ii) la mesure électrochimique du signal redox spécifique de ladite molécule d'intérêt.

On entend par « mesure électrochimique », la mesure d'une variation du potentiel d'oxydoréduction de la molécule redox ou la mesure d'une variation de type ampérométrique par variation du courant d'oxydation observé à un potentiel donné ou la mesure d'une variation d'impédance à un potentiel donné. Ces variations sont mesurées selon des méthodes bien connues de l'Homme du métier.

Lorsqu'une molécule d'intérêt est présente dans un échantillon, son interaction avec la sonde oligonucléotidique permet d'interrompre l'interaction entre ladite sonde oligonucléotidique et le nanomatériau portant des fonctions positives, afin de démasquer la molécule redox, créant ainsi un courant faradique.

La mesure électrochimique permet de détecter ce courant, représenté par un signal redox. Ce signal est par conséquent spécifique de ladite molécule d'intérêt.

La mesure électrochimique pour la mise en œuvre de l'invention peut être une mesure potentiométrique, une mesure d'impédancemétrie, une mesure coulométrique ou une mesure ampérométrique.

Dans un mode de réalisation avantageux, la mesure électrochimique est mise en œuvre par une mesure ampérométrique.

La mesure du courant électrique peut être réalisée au moyen des techniques ampérométriques connues, préférentiellement par une voltampérométrie à balayage de potentiel pouvant être linéaire, cyclique, à impulsion, ou encore du type à saut de potentiel, tel que la chronoampérométrie.

Selon l'invention, l'échantillon doit être à un pH pour lequel, au sein dudit système de détection, la sonde oligonucléotidique est chargée négativement et le nanomatériau est porteur de charges positives.

En particulier, l'échantillon doit être à un pH compris entre 6 et 7.

Conformément à l'invention, ledit échantillon peut être un échantillon biologique.

Avantageusement, cet échantillon peut avoir été prélevé sur un patient à des fins de diagnostic. L'échantillon peut être, par exemple, l'urine, le sang, le sérum, le plasma, des extraits cellulaires ou un fluide corporel.

Selon l'invention, la méthode de détection électrochimique peut comprendre, en plus des étapes (i) et (ii) et après l'étape (ii), le dosage quantitatif de ladite molécule d'intérêt. La quantification de la ou des molécule(s) d'intérêt se fait à l'aide d'une courbe de calibration établie au préalable selon l'une des méthodes connue de l'Homme du métier.

Selon un mode de réalisation particulier, l'invention concerne une méthode électrochimique de détection multiple d'au moins deux types de molécules d'intérêt.

Ladite méthode comprend les étapes suivantes :
(i) la mise en contact d'un système de détection comprenant au moins deux types de molécules redox et au moins deux types de sondes oligonucléotidiques, tels que décrits ci-dessus, avec un échantillon susceptible de contenir lesdites molécules d'intérêt ;
(ii) la mesure des signaux redox spécifiques auxdites molécules d'intérêt.
Ladite méthode permet de détecter simultanément au moins deux types de molécules d'intérêt.

La présente invention porte également sur un support modifié pour la préparation du système de détection.

Ledit support comprend :
- un matériau conducteur,
- un nanomatériau portant des fonctions positives, et
- au moins une molécule redox,
ledit nanomatériau étant situé entre ledit matériau conducteur et ladite molécule redox et étant lié de manière covalente à ces deux derniers.

Ledit support permet de fournir une plateforme prête à être fonctionnalisée par des sondes oligonucléotidiques selon les besoins de l'utilisateur.

Ledit support peut se lier, par l'intermédiaire de molécule redox fonctionnalisée, à un oligonucléotide dont l'extrémité 5' est également modifiée.

Ledit support peut être préparé selon la méthode suivante :
- recouvrir un matériau conducteur par un nanomatériau portant des fonctions positives, en formant des liaisons covalentes entre ledit matériau conducteur et ledit nanomatériau ;
- fonctionnaliser ledit nanomatériau portant des fonctions positives par au moins une molécule redox également fonctionnalisée.

L'invention prévoit également la méthode de préparation dudit système de détection électrochimique qui comprend :
(i) la mise en contact dudit support modifié tel que décrit ci-dessus avec au moins un oligonucléotide fonctionnalisé ciblant une molécule d'intérêt ;
(ii) la formation des liaisons covalentes entre le susdit support modifié et ledit oligonucléotide fonctionnalisé.

Lors de l'étape (ii), la formation de liaison covalente entre ledit support modifié et ledit oligonucléotide fonctionnalisé peut se faire aussi bien entre ledit oligonucléotide fonctionnalisé et ledit nanomatériau ou entre ledit oligonucléotide fonctionnalisé et ladite molécule redox.

La méthode de préparation dudit système de détection électrochimique selon l'invention, nécessite la fonctionnalisation préalable de la sonde oligonucléotidique à l'extrémité 3' ou 5' et la fonctionnalisation préalable de la molécule redox. Lorsqu'elles sont mises en contact, ledit oligonucléotide fonctionnalisé et la molécule redox forment des liaisons covalentes par l'intermédiaire de ces groupes fonctionnels.

On entend par « oligonucléotide fonctionnalisé » un oligonucléotide dont l'extrémité 5' ou 3' est modifiée par une fonction active, telle qu'une amine, un acide, un azide, un alcyne, un thiol, ... et susceptible de mettre en œuvre une réaction chimique rapide de type « chimie-click » avec un composé comportant une autre fonction active compatible.

Avantageusement, la présente invention propose également un kit de détection ultrasensible de molécules d'intérêt pouvant servir dans le diagnostic précoce ou ambulant.

Ledit kit comprend:
(i) un support modifié tel que décrit ci-dessus, et
(ii) au moins un oligonucléotide ciblant une molécule d'intérêt.

Ledit kit permet aux utilisateurs de préparer eux-mêmes à leur convenance un système de détection électrochimique selon l'invention et d'éviter le vieillissement ou la dégradation de la sonde oligonucléotidique qui puisse donner de faux négatifs. Afin de se lier à la molécule redox contenue dans ledit support modifié, ledit oligonucléotide est modifié à son extrémité 5' par ajout d'un groupement amine.

Selon un mode de réalisation de l'invention, le kit de préparation dudit système de détection électrochimique selon l'invention peut comprendre :
- au moins deux types de molécules redox dont les potentiels d'oxydoréduction sont distincts, et
- au moins deux types de sondes oligonucléotidiques simple brin, chaque type de sondes oligonucléotidiques étant lié à une molécule redox dont les potentiels d'oxydoréduction sont distincts les uns des autres.

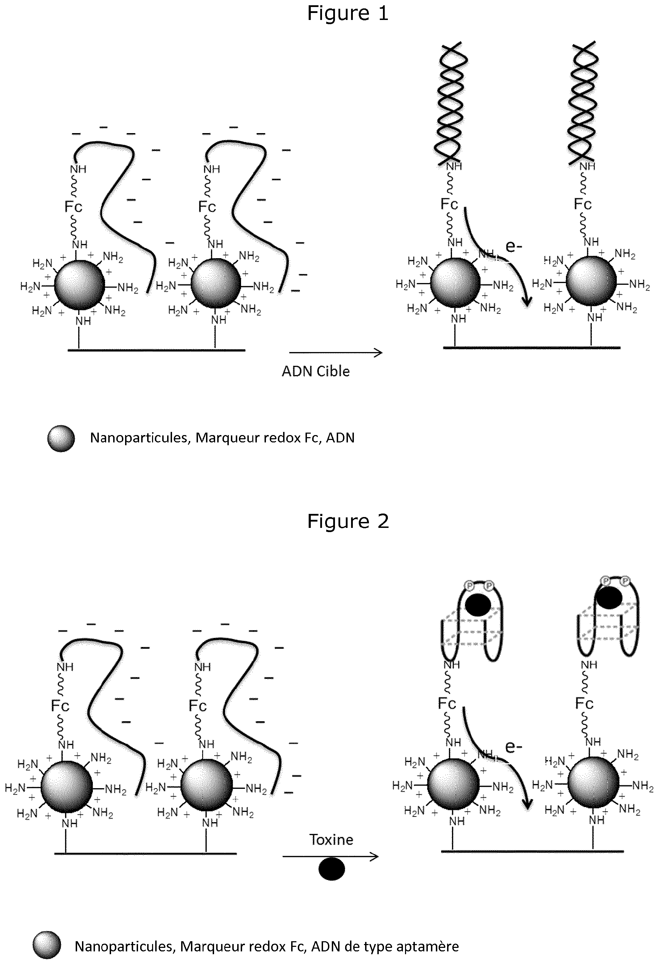

Les figures 1 à 10 et les exemples ci-après illustrent la présente invention.
La figure 1 représente le système de détection selon l'invention en absence et en présence d'une molécule d'intérêt, en particulier un ADN.
La figure 2 représente le système de détection selon l'invention en absence et en présence d'une molécule d'intérêt, en particulier un aptamère.
Les figures 3a et 3b présentent la spécificité du système de détection selon l'invention lorsque le nanomatériau utilisé est le PAMAM G4. La figure 3c présente la gamme de concentrations détectables dudit système.
Les figures 4a et 4b présentent la spécificité du système de détection selon l'invention lorsque le nanomatériau utilisé est des nanoparticules d'or modifiées. La figure 4c présente la gamme de concentrations détectables dudit système.
Les figures 5a et 5b présentent la spécificité du système de détection selon l'invention lorsque le nanomatériau utilisé est PAMAM G4 associé à des nanoparticules d'or modifiées. La figure 5c présente la gamme de concentrations détectables dudit système.
La figure 6 présente la spécificité du système de détection selon l'invention lorsque la sonde oligonucléotidique est un ADN synthétique, et en particulier dans la détection du virus de l'hépatite C.
Les figures 7a et 7b présentent la spécificité du système de détection selon l'invention lorsque la sonde oligonucléotidique est un aptamère spécifique de l'Ochratoxine A. En particulier, la figure 7b présente la sélectivité dudit système de détection pour l'Ochrotoxine A *vs* l'Ochratoxine B.
La figure 8 présente la spécificité du système de détection de l'Ochratoxine A selon l'invention dans lequel le nanomatériau utilisé est PAMAM G2 modifié avec naphtoquinone comme la sonde redox et l'aptamère spécifique à l'Ochratoxine A.
La figure 9 présente la spécificité du système de détection selon l'invention comportant des nanoparticules magnétiques d'oxyde de fer (Fe₃0₄) enrobées de chitosan et modifiées avec la sonde redox naphtoquinone pour la détection de l'Ochratoxine A.
La figure 10 présente la spécificité du système de détection selon l'invention comportant des microsphères de chitosan et modifiées avec la sonde redox naphtoquinone pour la détection de l'Ochratoxine A.

### EXEMPLES

### Matériels et méthodes

### - Produits

Le ferrocène Fc(NHP)₂ est synthétisé selon des méthodes connues de l'Homme du métier.

La solution saline de tampon phosphate (PBS) pH = 7,4 contient 10mM de Na₂HPO₄, 1,8mM de KH₂PO₄, 2,7mM de KCI et 137mM de NaCl. Le PBS est préparé avec de l'eau bidistillée et filtré au travers de membranes de 0,22µm puis stocké à 4°C jusqu'à son utilisation.

Les dendrimères PAMAM G4 sont purifiés par des membranes de filtration de 0,22µM avant utilisation.

Les nanoparticules d'or (AuNPs) proviennent de chez Sigma-Aldrich dans une solution de 0,1mM de PBS et ont un diamètre de 5nm.

La sonde oligonucléotidique utilisée pour étudier la gamme dynamique du système de l'invention, désignée ci-après par « sonde ADNss », comporte un oligonucléotide de séquence SEQ ID NO : 1 (5'GAT-ACT-TCT-ATC-ACC3'), qui est modifié à l'extrémité 5' par le groupement NH2C6-.

Ladite sonde cible un oligonucléotide d'intérêt synthétique de séquence SEQ ID NO : 2 (5'GGT- GAT-AGA-AGT-ATC3'), désigné ci-après ADN d'intérêt synthétique. Un oligonucléotide de séquence SEQ ID NO : 3 (5'CAT-TCC-CTC-TTA-GG3') non complémentaire avec la susdite sonde oligonucléotide est utilisé en tant que contrôle.

Dans le cadre d'expérience de détection de *Mycobacterium tuberculosis* résistant, un produit PCR de 411 bases est obtenu à partir du gène rpoB de *Mycobacterium tuberculosis.*

cPCR est le produit PCR obtenu de 5 souches de *Mycobacterium tuberculosis* comportant la mutation TCG/TTG dans le gène rpoB qui est responsable de la résistance à la rifampicine. Ces 5 souches sont 2-09, 7-09, 8-09, 10-09 et 11-09.

ncPCR est le produit PCR obtenu à partir du gène rpoB non-muté des souches sauvages de *Mycobacterium tuberculosis.*

La sonde oligonucléotidique ciblant cPCR, désignée ci-après par « sonde PCR », comporte un oligonucléotide de séquence SEQ ID NO : 4 (5'CCG-ACT-GTT-GGC-GCT-GGG3'), dont l'extrémité 5' est modifiée par le groupement NH₂C₆- pour la mise en œuvre de la liaison covalente avec le ferrocène.

Dans le cadre des expériences de détection des virus de l'hépatite C, la sonde oligonucléotidique ciblant le virus comporte un oligonucléotide de séquence SEQ ID NO : 5 (5TCA ACT TCG GGA ATC TCA ATG TTA G3'), dont l'extrémité 5' est modifiée par le groupement NH₂C₆- pour la mise en œuvre de la liaison covalente avec le ferrocène.

Dans le cadre d'expérience de détection de l'Ochratoxine (OTA), la sonde ciblant la toxine est une sonde de type aptamère de séquence SEQ ID NO : 6 (5'GAT CGG GTG TGG GTG GCG TAA AGG GAG CAT CGG ACA3'), dont l'extrémité 5' est modifiée par le groupement NH₂C₆- pour la mise en œuvre de la liaison covalente avec le ferrocène.

### - Techniques de mesure

### Voltampérométrie cyclique (CV)

Les analyses sont faites dans 0,1M de KCI avec le couple [Fe(CN)₆]⁴⁻5mM/[Fe(CN)₆]³⁻ 5mM dans une gamme de potentiels de -0,2 et 0,5V avec un taux de balayage de 50mV.s⁻¹.

### Spectrométrie d'impédance (EIS)

Les analyses sont faites dans 0,1M de KCI avec le couple [Fe(CN)₆]⁴⁻ 5mM /[Fe(CN)₆]³⁻ 5mM. Toutes les impédances sont obtenues à 0,2V vs. Ag/AgCl à un potentiel DC de 10mV avec une gamme de fréquences de 100KHz à 0,1Hz.

### Voltammétrie à vague carré (SWV)

Les analyses sont faites dans du PBS pH = 7,4 contenant 10mM de Na₂HPO₄, 1,8mM de KH₂PO₄, 2,7mM de KCI et 137mM de NaCl filtré par des membranes de 0,22µm et stocké à 4°C jusqu'à utilisation. SWV sont effectués dans une gamme de potentiels de -0,3 à 0,4 avec un temps de conditionnement de 180s, de fréquence 25Hz et d'amplitude de modulation de 20mV.

### Voltampérométrie à impulsion différentielle (DPV)

Les analyses sont effectuées dans du PBS pH = 7,4 contenant 10mM de Na₂HPO₄, 1,8mM de KH₂PO₄, 2,7mM de KCI et 137mM de NaCl filtré par des membranes de 0,22µm et stocké à 4°C jusqu'à utilisation. Les analyses DPV sont effectuées dans une gamme de potentiels de -0,3 à 0,4V à un taux de balayage de 50mV.s⁻¹ avec une hauteur d'impulsion de 45mV et 0,05s de largeur d'impulsion.

### Exemple 1 : Système de détection comportant PAMAM G4

### - Modification de la surface de l'électrode en carbone vitreux avec les dendrimères PAMAM G4

Les liaisons covalentes des dendrimères PAMAM G4 sur l'électrode de carbone vitreux sont faites dans de l'eau contenant 0,5M de LiClO₄ par méthode de CV en balayant le potentiel de 0,0 à 1,1V vs. Ag/AgCl en tant qu'électrode de référence pendant un cycle avec une vitesse de balayage de 50mV.s⁻¹. Pendant la réaction, l'électrode de travail et la contre-électrode sont séparées dans des cellules de faible volume (Basi) contenant 200µl d'une solution de PAMAM G4 de 50µM. Après la fixation des molécules à la surface et un lavage délicat des électrodes à l'eau bidistillée, les analyses de CV et d'EIS sont réalisées.

### - Association de la molécule redox ferrocène et l'oligonucléotide modifié de séquence SEQ ID NO : 1

Le ferrocène modifié par deux groupes phtalymidyl Fc(NHP)₂ est associé aux dendrimères dont la surface est modifiée. La réaction se fait par immersion de l'électrode dans une solution d'1mM de ferrocène dans de l'acétonitrile pendant 1h à température ambiante. Les résidus non liés sont lavés à l'acétonitrile et à l'eau bidistillée. Puis l'électrode est immergée dans une solution de 10µM de sondes ADNss-C₆NH₂ de SEQ ID NO : 1 modifiées préparées dans du PBS pendant 1h à température ambiante. L'électrode est ensuite rincée dans l'eau distillée et du PBS, et afin de saturer les esters phtalymidyle de ferrocène, elle est immergée dans une solution de 1mM d'éthanolamine en solution de PBS pendant 30min à température ambiante. Le système de détection est ensuite lavé à l'eau distillée et du PBS puis stocké dans du PBS à 4°C jusqu'à utilisation. Après chaque étape de construction du système de détection à ADN, les modifications de surface sont contrôlées par méthode SWV.

### - Hybridation avec l'oligonucléotide complémentaire de SEQ ID NO : 2

L'hybridation des 15 bases complémentaires à la sonde d'ADNss de SEQ ID NO : 1 associée à la surface est effectuée par immersion de l'électrode dans la solution de l'ADN synthétique (SEQ ID NO : 2) pendant 1h à 40°C. Les différentes concentrations utilisées pour l'hybridation sont 100fM, 1pM, 10pM, 100pM, 1nM, 10nM et 100nM. Après chaque incubation, l'électrode est lavée délicatement avec de l'eau distillée et du PBS puis l'électrode est analysée par méthode SWV. Le test de contrôle est effectué par immersion de l'électrode dans 1µM d'oligonucléotide non-complémentaire (SEQ ID NO : 3) dans du PBS et incubation dans les mêmes conditions que pour l'ADN d'intérêt synthétique (SEQ ID NO : 2) (1h à 40°C). Les mêmes conditions sont appliquées pour détecter les échantillons issus de PCR qui sont préalablement traités à 90°C pendant 5min pour déshybridation des ADN double-brins obtenus à l'issu de la PCR.

### Résultats

### a) Détection de l'ADN d'intérêt synthétique de séquence SEQ ID NO : 2

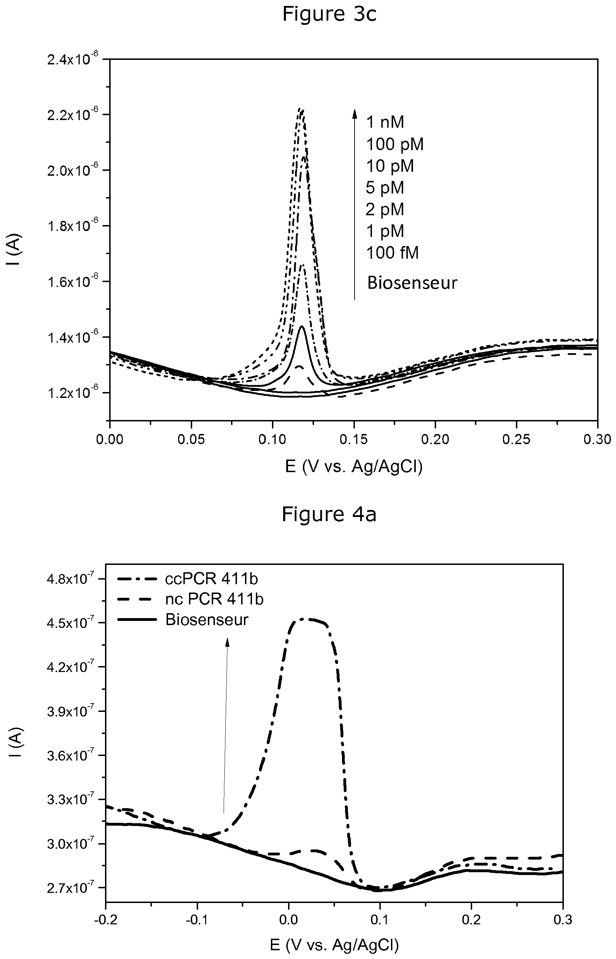

La détection de l'ADN d'intérêt synthétique de séquence SEQ ID NO : 2 est effectuée par méthode SWV. Le système de détection est incubé successivement dans une solution contenant des concentrations d'ADN variables entre 100fM et 1nM et une analyse par SWV est effectuée pour chaque concentration. La figure 3c montre l'augmentation du courant correspondant au ferrocène à 0,11V avec chaque concentration d'ADN d'intérêt. Cette variation de courant est proportionnelle à la concentration d'ADN d'intérêt synthétique.

Le premier signal positif pendant la détection d'ADN d'intérêt est obtenu après incubation du système de détection dans une solution d'ADN d'intérêt de 1pM et entraine une variation de courant de 10%. Et, la saturation du système de détection est observée à 100pM avec une variation de courant de 97% ce qui correspond aux changements du signal redox du ferrocène.

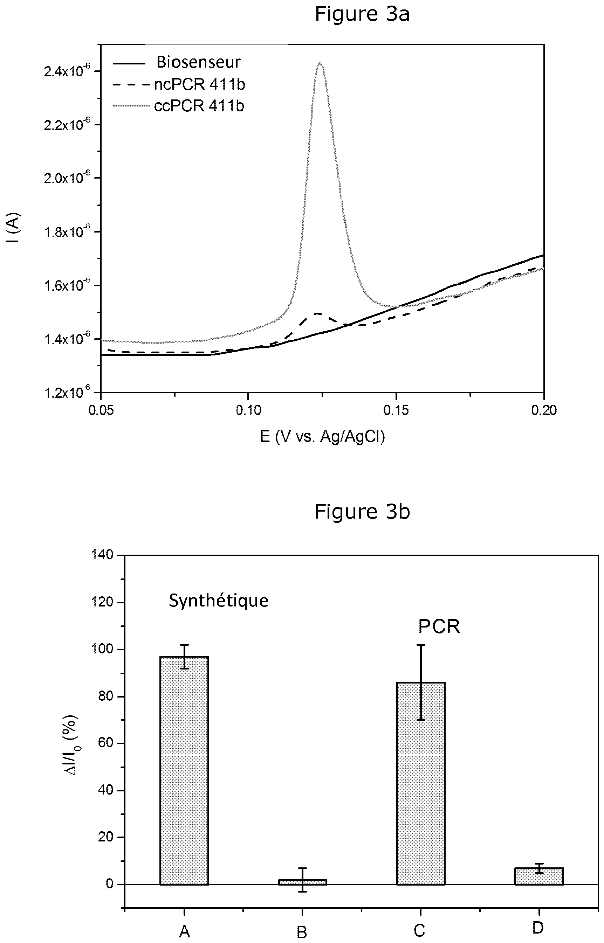

L'augmentation du courant lors de la détection de l'ADN d'intérêt s'explique par le dévoilement de la molécule redox par l'hybridation de l'ADN d'intérêt complémentaire synthétique (SEQ ID NO : 2) à la sonde oligonucléotidique SEQ ID NO : 1 et par l'utilisation de PAMAM G4. Ce changement d'orientation du complexe sonde ADNss/ADN d'intérêt complémentaire synthétique ou issu d'échantillons PCR induit l'augmentation du transfert d'électrons entre le ferrocène et le PAMAM G4 (figures 3a et 3c).

### b) Détection de l'ADN d'intérêt issu de la PCR

Le système de détection comportant PAMAM G4 et la sonde oligonucléotidique de séquence SEQ ID NO : 4 est préparé selon les méthodes décrites ci-dessus pour détecter l'ADN d'intérêt composé de 411 bases issu de PCR (cPCR).

Après l'incubation, la variation de courant correspondant au ferrocène est calculée. La valeur de la variation obtenue par 5 expériences indépendantes est de 86%. Lorsque l'échantillon ne contient le produit ncPCR dont la séquence diffère de celle de cPCR par un acide nucléotidique, la variation de courant n'est plus que de 7% (figure 3a et figure 3b). Ces résultats montrent que ledit système peut détecter efficacement et spécifiquement un ADN d'intérêt comme celui de *Mycobacterium tuberculosis* et différencier les souches résistantes à la rifampicine. Il peut être utilisé dans le domaine clinique.

### Exemple 2 : Système de détection comportant des nanoparticules d'or

### - Modification des nanoparticules d'or et leur association à la surface

2g (0,008mol) de dichlorhydrate de cystamine sont dissous dans 20ml de NaOH 5M (0,1M) et extrait avec 10ml de CH₂Cl₂. La phase organique est rincée avec 10ml d'eau distillée, séchée sur du MgSO₄ et concentrée sous vide. De la cystamine dissoute dans de l'eau bidistillée est ajoutée à la solution de 1ml de nanoparticules d'or (DO=0,4) pour une concentration finale de 50µM. La solution est incubée à 4°C jusqu'à utilisation. La concentration en cystamine ainsi que le temps d'incubation ont été optimisé pour permettre d'obtenir des puits de nanoparticules d'or modifiées non agrégées afin d'être analysés par spectrométrie ultraviolet-visible et diffusion dynamique de la lumière.

Après cette réaction, l'échantillon de nanoparticules d'or modifiées avec la cystamine (AuNPs-NH₂) est centrifugé pendant 20min à 14 000 tours/min pour éliminer les résidus cystamine. Le culot est ensuite rincé deux fois avec 1ml d'eau bidistillée et centrifugé jusqu'à ce que l'excès de cystamine soit éliminé. Au final, l'échantillon d'AuNPs-NH₂ est dissous dans 300µl d'eau.

100µl d'AuNPs-NH₂ sont mélangés avec une solution de 100µl de N-(3-diméthylaminopropyl)-N'-éthylcarbodiimide hydrochloride/N-Hydroxysuccinimide (10mM/10mM dissous dans l'eau) afin d'associer les AuNPs-NH₂ à l'électrode de carbone vitreux. Puis, l'électrode modifiée avec des groupes carboxyliques -COOH est immergée dans ce mélange et est incubée pendant 2h à température ambiante. Différents temps d'incubation ont été testés et 2h est la durée optimale pour une bonne performance du système de détection. L'électrode est rincée délicatement avec de l'eau bidistillée puis est analysée par mesures EIS.

La modification d'AuNPs par la cystamine est optimisée et contrôlée par spectrométrie ultraviolet-visible. Les AuNPs de 5nm de diamètre absorbent les ultraviolets visibles à 520nm, tandis que la valeur d'absorption des AuNPs modifiés est augmentée et confirme ainsi l'efficacité de la réaction.

### - Electrodéposition de β-alanine et d'éthanolamine

La liaison covalente de la β-alanine sur l'électrode de carbone vitreux est effectuée dans de l'eau contenant 0,5M de LiClO₄ par balayage des potentiels de 0,2 à 1,1V vs. Ag/AgCl comme électrode de référence pendant 10 cycles jusqu'à saturation avec taux de balayage de 50mV.s⁻¹. Pendant la réaction l'électrode de travail et la contre-électrode sont séparées dans des cellules de faible volume (Basi) contenant 200µl d'une solution de 10mM de β-alanine. Puis l'électrode est rincée délicatement à l'eau bidistillée. La saturation de la surface par l'éthanolamine est effectuée dans les mêmes conditions, par balayage des potentiels de 0,2 à 1,1V vs. Ag/AgCl pendant 3 cycles. La concentration d'éthanolamine utilisée pour cette réaction est d'1mM préparé dans de l'eau contenant 0,5M de LiClO₄. Après la fixation des molécules à la surface et le rinçage délicat de l'électrode à l'eau bidistillée, les mesures EIS sont effectuées.

### - Construction du système de détection

Le ferrocène modifié par deux groupes phtalymidyl Fc(NHP)₂ est associé à la surface AuNPs-NH₂ modifiée. La réaction est effectuée par immersion de l'électrode dans une solution d'1mM de ferrocène dans l'acétonitrile pendant 1h à température ambiante. Les résidus non liés sont évacués par rinçage à l'acétonitrile et à l'eau bidistillée. Puis, l'électrode est immergée dans une solution de 10µM d'ADN SEQ ID NO : 1 modifiée préparée dans du PBS pendant 1h à température ambiante. Après le rinçage de l'électrode à l'eau distillée et au PBS, l'électrode est immergée dans une solution d'1mM d'éthanolamine dans du PBS pendant 30min à température ambiante afin de saturer les esters phtalymidyle de ferrocène. Le système de détection est ensuite lavé avec de l'eau distillée et du PBS puis stocké dans du PBS à 4°C jusqu'à utilisation. Après chaque étape de construction du senseur à ADN, les modifications de surface sont contrôlées par la méthode EIS.

### - Hybridation avec l'ADN d'intérêt complémentaire synthétique

L'hybridation de l'ADN d'intérêt synthétique complémentaire à la sonde ADNss associée à la surface est effectuée par immersion de l'électrode dans la solution de l'ADN d'intérêt synthétique de SEQ ID NO : 2 pendant 1h à 40°C. Les différentes concentrations utilisées pour l'hybridation sont 100fM, 1pM, 10pM, 100pM, 1nM, 10nM et 100nM. Après chaque incubation, l'électrode est lavée délicatement avec de l'eau distillée et du PBS puis l'électrode est analysée par la méthode DPV.

Les produits de PCR contiennent 411 bases et sont détectés par un système de détection comportant la sonde PCR de séquence SEQ ID NO : 4 dans une solution selon le même procédé décrit ci-dessus.

Le test de contrôle est effectué par immersion de l'électrode dans 1µM de cible non-complémentaire dans du PBS et incubé dans les mêmes conditions que pour les ADN d'intérêt complémentaires synthétique (1h à 40°C). Les mêmes conditions sont appliquées pour détecter les échantillons issus de PCR.

### Résultats

### a) Détection de l'ADN d'intérêt synthétique de séquence SEQ ID NO : 2

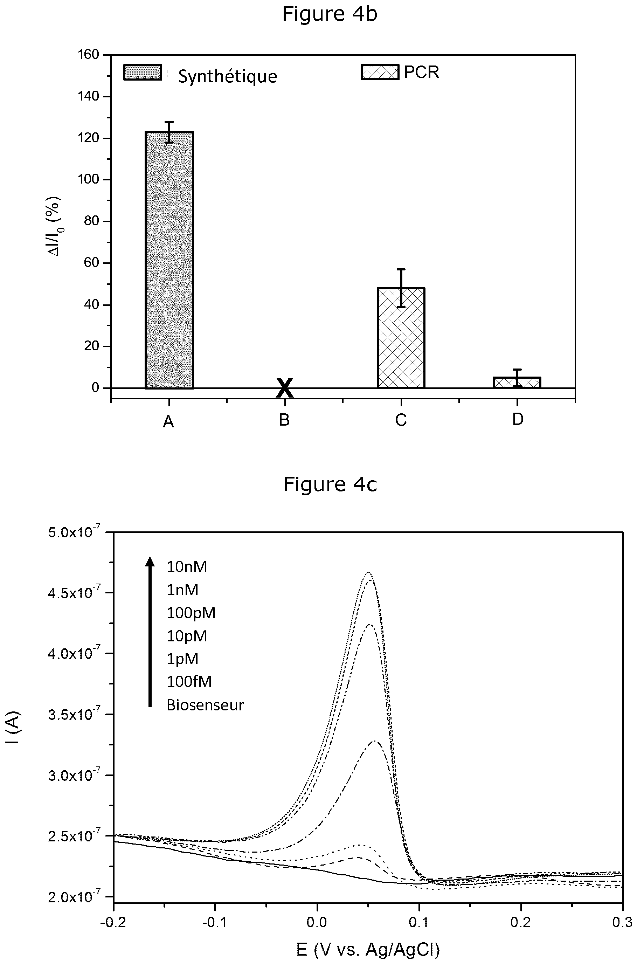

La détection de l'ADN d'intérêt est effectuée par la méthode SWV. Le système de détection est incubé successivement dans une solution contenant des concentrations d'ADN variables entre 100fM et 1nM et une analyse par SWV est effectuée pour chaque concentration. La figure 4c montre l'augmentation du courant correspondant au ferrocène à 0,11V avec chaque concentration d'ADN d'intérêt complémentaire. Cette variation de courant est proportionnelle à la concentration d'ADN d'intérêt synthétique (SEQ ID NO : 2).

Le premier signal positif pendant la détection d'ADN d'intérêt synthétique est obtenu après incubation du système de détection dans une solution contenant 100fM d'ADN d'intérêt synthétique et entraine une variation de courant de 10%.

Et, la saturation du système de détection est observée à 100pM avec une variation de courant de 97% ce qui correspond aux changements du ferrocène. Néanmoins, des analyses à des concentrations de 100pM à 1nM sont réalisables avec le système de détection utilisé (Figure 4c).

b) Le même système de détection que celui mentionné dans l'exemple 2a) est construit avec des sondes oligonucléotidiques de séquence SEQ ID NO : 4 pour détecter un échantillon comprenant le produit de PCR correspondant au gène rpoB muté (cPCR) de *Mycobacterium tuberculosis.*

Ledit système de détection est capable de donner un signal électrique spécifique lorsque le produit cPCR est présent dans l'échantillon (Figures 4a et 4b). Ledit système permet de détecter de façon spécifique les souches de *Mycobacterium tuberculosis* résistantes à la rifampicine.

### Exemple 3 : Système de détection comportant des nanoparticules d'or et PAMAM G4 (G4-AuNPs)

### - Modification de la surface de l'électrode en carbone vitreux avec les dendrimères PAMAM G4

Les liaisons covalentes des dendrimères PAMAM G4 sur l'électrode de carbone vitreux sont faites dans de l'eau contenant 0,5M de LiClO₄ par méthode de CV en balayant le potentiel de 0,0 à 1,1V vs. Ag/AgCl en tant qu'électrode de référence pendant un cycle avec un taux balayage de 50mV.s⁻¹. Pendant la réaction, l'électrode de travail et la contre-électrode sont séparées dans des cellules de faible volume (Basi) contenant 200µl d'une solution de PAMAM G4 de 50µM. Après la fixation des molécules à la surface et un lavage délicat des électrodes à l'eau bidistillée, les analyses de CV et d'EIS sont réalisées.

### - Modification de la surface de l'électrode en carbone vitreux avec les dendrimères PAMAM G4 et les AuNPs

La première étape dans la construction du système de détection est l'électrodéposition des dendrimères PAMAM G4 à la surface de l'électrode en carbone vitreux. L'électrodéposition est faite par la technique de CV en balayant le potentiel de 0,0 à 1,1V vs. Ag/AgCl en tant qu'électrode de référence pendant un cycle avec une vitesse de balayage de 50mV.s⁻¹. Pendant la réaction d'oxydation, les groupes amines des dendrimères forment un radical cationique -NH•⁺ qui interagit avec les groupes aromatiques C=C de la surface carbonée.

### - Modification des nanoparticules d'or et leur association à la surface

2g (0,013mol) de dichlorhydrate de cystamine est dissous dans 20 ml de NaOH 5M (0.1M) et extrait avec 10ml de CH₂Cl₂. La couche organique est rincée avec 10ml d'eau distillée, séchée sur du MgSO₄ et concentrée sous vide. De la cystamine dissoute dans de l'eau bidistillée est ajoutée à la solution de 1ml de nanoparticules d'or (DO=0,4) pour une concentration finale de 50µM. La solution est incubée à 4°C jusqu'à utilisation.

Après cette réaction, l'échantillon de nanoparticules d'or modifiées avec la cystamine (AuNPs-NH₂) est centrifugé pendant 20min à 14000 tours/min pour éliminer les résidus cystamine. Le culot est ensuite rincé deux fois avec 1ml d'eau bidistillée et centrifugé jusqu'à ce que l'excès de cystamine soit éliminé. Au final, l'échantillon d'AuNPs-NH₂ est dissous dans 300µl d'eau.

### - Association des AuNPs et des dendrimères PAMAM G4

Afin d'associer les AuNPs-NH₂ à l'électrode de carbone vitreux modifiée par les dendrimères PAMAM G4, le glutaraldéhyde est utilisé comme lien. L'électrode modifiée par les dendrimères PAMAM G4 est immergée dans une solution de 0,5% de glutaraldéhyde pendant 20min puis est rincée délicatement. Ensuite, l'électrode est incubée dans 100µl d'AuNPs-NH₂ pendant 2h à température ambiante. Différents temps d'incubation ont été testés et 2h est la durée optimale pour une bonne performance du système de détection. L'électrode est rincée délicatement avec de l'eau bidistillée et est ensuite analysée par mesures CV et EIS.

### - Association de la molécule redox ferrocène et la sonde ADNss

Le ferrocène modifié par deux groupes phtalymidyl Fc(NHP)₂ est associé aux AuNPs-NH₂ dont la surface est modifiée. La réaction se fait par immersion de l'électrode dans une solution d'1mM de ferrocène dans de l'acétonitrile pendant 1h à température ambiante. Les résidus non liés sont lavés à l'acétonitrile et à l'eau bidistillée. Puis l'électrode est immergée dans une solution de 10µM de sondes ADNss-C₆NH₂ préparées dans du PBS pendant 1h à température ambiante. L'électrode est ensuite rincée dans l'eau distillée et du PBS, et immergée pendant 30min à température ambiante dans une solution de 1mM d'éthanolamine en solution de PBS afin de saturer les esters phtalymidyle de ferrocène. Le système de détection est ensuite lavé avec de l'eau distillée et du PBS puis stocké dans du PBS à 4°C jusqu'à utilisation. Après chaque étape de construction du système de détection à ADN, les modifications de surface sont contrôlées par la méthode SWV.

### - Hybridation avec l'ADN d'intérêt synthétique de séquence SEQ ID NO : 2

L'hybridation de l'ADN d'intérêt synthétique (SEQ ID NO : 2) à la sonde d'ADNss SEQ ID NO : 1 associée à la surface est effectuée par immersion de l'électrode dans la solution de l'ADN d'intérêt synthétique pendant 1h à 40°C. Les différentes concentrations utilisées pour l'hybridation sont 100fM, 1pM, 10pM, 100pM, 1nM, 10nM et 100nM. Après chaque incubation, l'électrode est lavée délicatement avec de l'eau distillée et du PBS puis l'électrode est analysée par la méthode SWV. Le test de contrôle est effectué par immersion de l'électrode dans 1µM d'ADN d'intérêt non-complémentaire de séquence SEQ ID NO : 3 dans du PBS et incubé dans les mêmes conditions que pour les ADN d'intérêt (1h à 40°C). Les mêmes conditions sont appliquées pour détecter les échantillons issus de PCR.

### Résultats

### a) Détection de l'ADN d'intérêt synthétique

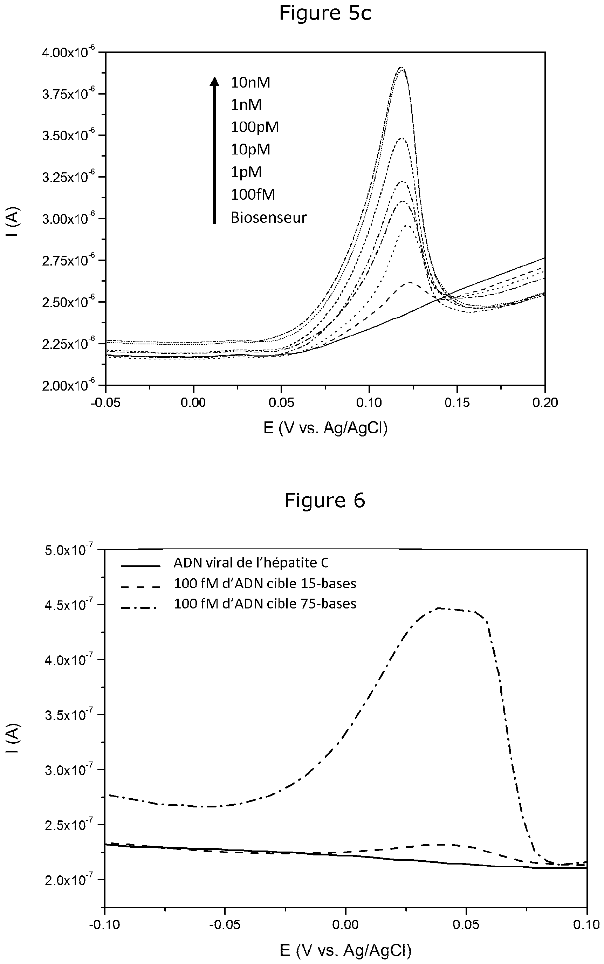

La détection de l'ADN d'intérêt SEQ ID NO : 2 est effectuée par la méthode SWV. Le système de détection est incubé successivement dans une solution contenant des concentrations d'ADN variables entre 100fM et 1nM et une analyse par SWV est effectuée pour chaque concentration. La figure 5c montre l'augmentation du courant correspondant au ferrocène à 0,11V avec chaque concentration d'ADN d'intérêt synthétique SEQ ID NO : 2. Cette variation de courant est proportionnelle à la concentration de l'ADN d'intérêt synthétique.

Le premier signal positif pendant la détection d'ADN d'intérêt SEQ ID NO : 2 est obtenu après incubation du système de détection dans une solution d'ADN d'intérêt SEQ ID NO : 2 de 1pM et entraine une variation de courant de 10%.

Et, la saturation du système de détection est observée à 100pM avec une variation de courant de 97% ce qui correspond aux changements du ferrocène. Néanmoins, des analyses à des concentrations de 100pM à 1nM sont faisables avec le système de détection utilisé (figure 5c).

b) Le même système de détection que celui mentionné dans l'exemple 2 est construit avec des sondes oligonucléotidiques de séquence SEQ ID NO : 4 pour détecter un échantillon comprenant le produit de PCR correspondant au gène rpoB muté (cPCR) de *Mycobacterium tuberculosis.*

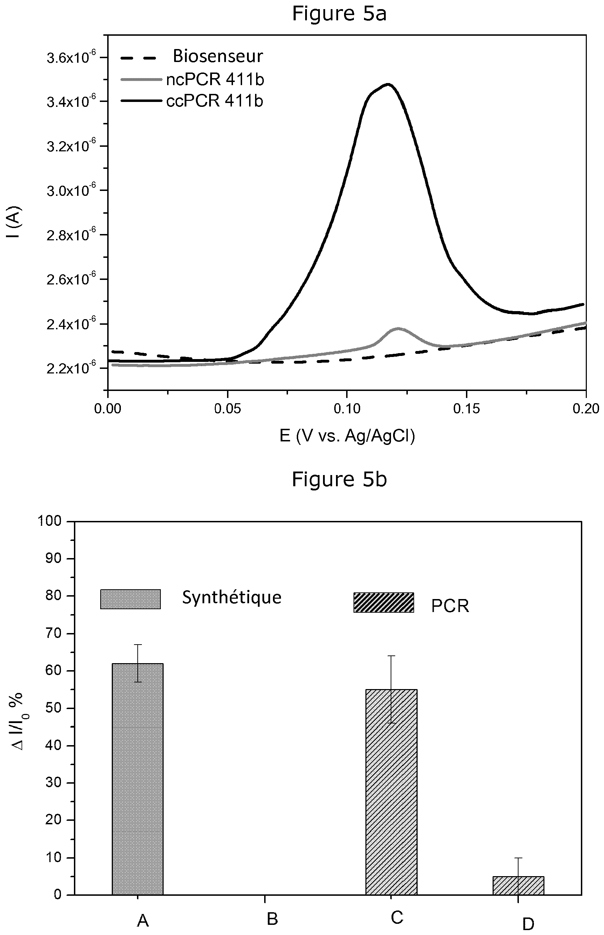

Ledit système de détection est capable de donner un signal électrique spécifique lorsque le produit cPCR est présent dans l'échantillon (Figures 5a et 5b).

c) Le même système de détection que celui mentionné dans l'exemple 3a) est construit avec des sondes oligonucléotidiques de séquence SEQ ID NO : 5 pour détecter un échantillon d'ADN synthétique du virus de l'hépatite C.

Ledit système de détection est capable de donner un signal électrique en présence de l'ADN synthétique (Figure 6). Ledit système permet donc de détecter le virus de l'hépatite C.

Le tableau I ci-dessous résume les gammes de détection obtenues selon les différents exemples cités ci-dessus :

**Tableau I**

| **Système de détection** | **Gamme dynamique** | **Gamme linéaire** | **Limite quantitative** |
|---|---|---|---|
| PAMAM | 1pM - 100pM | 1pM - 10pM | 1pM |
| AuNPs | 100fM - 1nM | 1pM - 1nM | 100fM |
| PAMAM-AuNPs | 100fM - 10nM | 100fM - 10nM | 100fM |

### Exemple 4 : Détection de l'ochratoxine A (OTA) avec un système de détection comportant PAMAM G4

Le même système de détection que celui mentionné dans l'exemple 1 est construit avec une sonde aptamère de séquence SEQ ID NO : 6 pour détecter la présence ou l'absence de l'OTA dans un échantillon alimentaire comme le lait ou le vin dont la matrice est complexe.

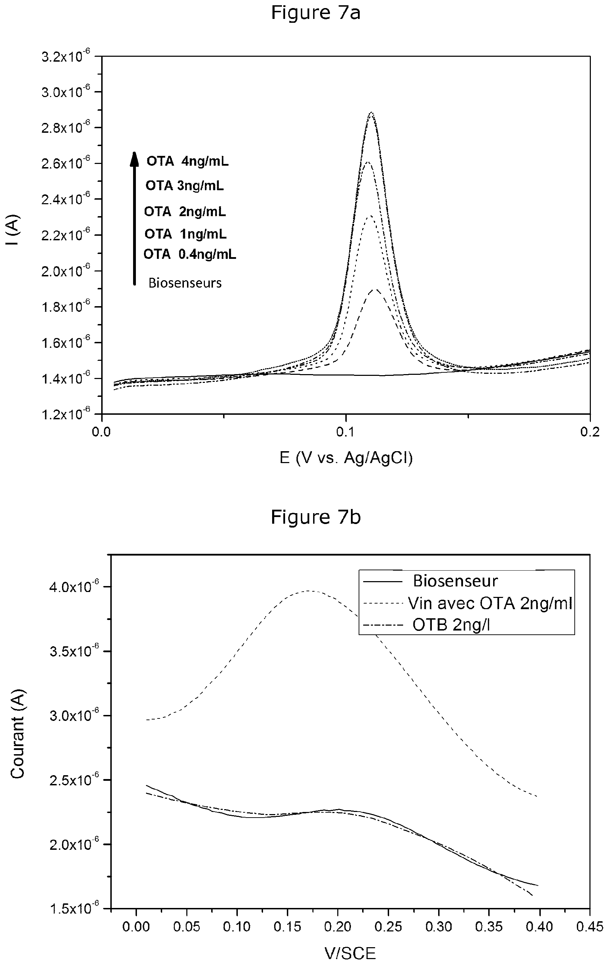

Ledit système de détection est capable de donner un signal électrique en présence de l'OTA (Figures 7a et 7b). Ledit système permet donc la détection de la toxine OTA. La présence d'une autre toxine non spécifique de l'aptamère tel que l'OTB qui n'est pas détectée (Figure 7b).

### Exemple 5 : Détection de l'OTA avec un système de détection comportant PAMAM G2

### - Modification de la surface de l'électrode en carbone vitreux avec les dendrimères PAMAM G2

Les liaisons covalentes des dendrimères PAMAM G2 sur l'électrode de carbone vitreux sont faites dans de l'eau contenant 0,5M de LiClO₄ par méthode de CV en balayant le potentiel de 0,0 à +1,1V vs. Ag/AgCl en tant qu'électrode de référence pendant un cycle avec une vitesse de balayage de 50mV.s⁻¹. Pendant la réaction, l'électrode de travail et la contre-électrode sont séparées dans des cellules de faible volume (Basi) contenant 200µl d'une solution de PAMAM G2 de 50µM. Après la fixation des molécules à la surface et un lavage délicat des électrodes à l'eau bidistillée, les analyses de CV et d'EIS sont réalisées.

### - Association de la molécule redox naphtoquinone et du PAMAM G2

La naphtoquinone modifiée par l'acide propanoïque est associée aux dendrimères. La réaction se fait par couplage peptidique entre l'acide propanoïque et les fonctions amines à la surface du dendrimère à l'aide du 1-Éthyl-3-(3-diméthylaminopropyl)carbodiimide (EDC) comme agent de couplage.

Afin de vérifier le bon greffage des molécules redox à la surface des dendrimères, le signal est analysé et présente un pic intense au potentiel de la molécule redox (-0,22V) immobilisée à la surface.

### - Association de la sonde oligonucléotidique modifiée de séquence SEQ ID NO : 6 et du PAMAM G2

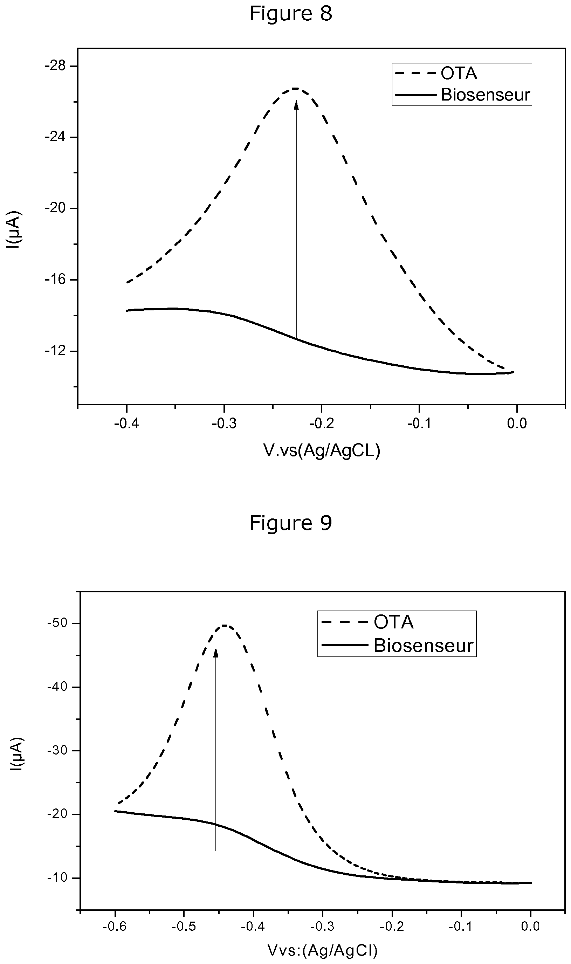

Par la suite l'électrode est incubée dans une solution d'ADN aptamère spécifique à l'OTA modifié par un groupe COOH dans sa position terminale5'. L'aptamère est greffé covalemment à la surface du dendrimère par couplage peptidique entre l'amine de la surface et la fonction acide en position 5' de l'aptamère ; cette réaction dure 1h. La surface est lavée puis stabilisé dans une solution tampon de PBS et le signal est mesuré (Figure 8).

Afin de vérifier le bon greffage des sondes oligonucléotidiques de SEQ ID NO : 6 sous forme d'aptamère à la surface des dendrimères, le signal émis par la molécule redox est analysé et présente une forte diminution au potentiel de la molécule redox (-0,22V) immobilisée à la surface.

### - Mise en contact de l'OTA avec le système de détection comportant du PAMAM G2

L'OTA est mis en contact avec le système de détection tel que préparé pendant 1h, puis la surface est lavée.

### Résultats

Le système de détection décrit ci-dessus est utilisé pour détecter la présence ou l'absence de l'OTA dans un échantillon alimentaire comme le lait ou le vin dont la matrice est complexe.

L'analyse du signal de la naphtoquinone (Figure 8) indique une augmentation du signal au potentiel de -0,22V, celle-ci étant due au démasquage de la molécule redox engendré par la liaison complémentaire entre l'OTA et son aptamère complémentaire de SEQ ID NO :6.

Autrement dit, ledit système de détection comportant PAMAM G2 est capable de donner un signal électrique en présence de la toxine OTA.

### Exemple 6 : Détection de l'OTA avec un système de détection comportant des nanoparticules magnétiques

Le même système de détection que celui mentionné dans l'exemple 5 est construit avec des nanoparticules magnétiques modifiées par du chitosan comme nanomatériau pour détecter la présence ou l'absence de l'OTA dans un échantillon alimentaire comme le lait ou le vin dont la matrice est complexe.

Les nanoparticules magnétiques sont des nanoparticules magnétiques d'oxyde de fer (Fe₃O₄) de taille 10nm-100nm et sont obtenues avec une synthèse classique puis modifiées par du chitosan pour le rendre cationique.

L'analyse du signal de la naphtoquinone (Figure 9) indique une augmentation du signal au potentiel de -0,4V, celle-ci étant due au démasquage de la molécule redox engendré par la liaison complémentaire entre l'OTA et son aptamère complémentaire de SEQ ID NO :6.

Autrement dit, ledit système de détection comportant des nanoparticules magnétiques modifiées avec du chitosan est capable de donner un signal électrique en présence de la toxine OTA.

### Exemple 7 : Détection de l'OTA avec un système de détection comportant des nanoparticules de chitosan

Le même système de détection que celui mentionné dans l'exemple 6 est construit avec des microsphères de chitosan comme nanomatériau pour détecter la présence ou l'absence de l'OTA dans un échantillon alimentaire comme le lait ou le vin dont la matrice est complexe.

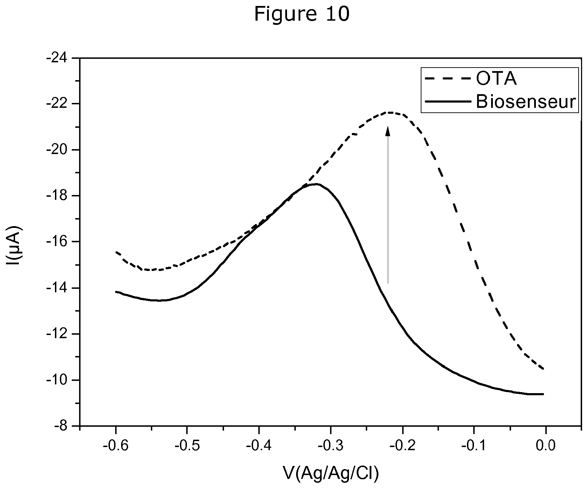

L'analyse du signal de la naphtoquinone (Figure 10) indique une augmentation du signal au potentiel de -0,22V, celle-ci étant due au démasquage de la molécule redox engendré par la liaison complémentaire entre l'OTA et son aptamère complémentaire de SEQ ID NO :6.

Autrement dit, ledit système de détection comportant des microsphères de chitosan est capable de donner un signal électrique en présence de la toxine OTA malgré une variation du signal moins importante que précédemment.

Cependant l'optimisation de la taille de ces microsphères de chitosan permettrait une plus grande variation du signal.

## Revendications

1. Système de détection électrochimique de molécules d'intérêt, **caractérisé en ce que** ledit système comprend :
- un matériau conducteur,
- au moins un nanomatériau portant des fonctions positives, lié covalemment audit matériau conducteur,
- au moins une molécule redox, et
- au moins une sonde oligonucléotidique simple brin ciblant une molécule d'intérêt,
ladite molécule redox étant liée covalemment audit nanomatériau ; ladite sonde oligonucléotidique étant liée covalemment audit nanomatériau ou à la molécule redox ;
le nombre de fonctions positives dudit nanomatériau étant supérieur au nombre de sondes oligonucléotidiques.

2. Système de détection électrochimique selon la revendication 1, **caractérisé en ce que** ladite molécule redox est liée covalemment à la fois audit nanomatériau et à la sonde oligonucléotidique.

3. Système de détection électrochimique selon la revendication 2, **caractérisé en ce que** ledit système comprend :
- au moins deux types de molécules redox dont les potentiels d'oxydoréduction sont distincts, et
- au moins deux types de sondes oligonucléotidiques en simple brin chacun ciblant une molécule d'intérêt différente,
le même type de sondes oligonucléotidiques étant lié au même type de molécules redox.

4. Système de détection électrochimique selon la revendication 1, **caractérisé en ce que** ladite sonde oligonucléotidique et ladite molécule redox sont respectivement liées covalemment à audit nanomatériau.

5. Système de détection électrochimique selon l'une quelconque des revendications précédentes, **caractérisé en ce que** ledit nanomatériau portant des fonctions positives est choisi parmi le groupe comprenant
- un dendrimère, préférentiellement un dendrimère de type de poly(amidoamine) notamment de deuxième, quatrième ou sixième génération,
- des particules métalliques comme des nanoparticules d'or,
- des nanoparticules magnétiques, telles que l'oxyde de fer,
- des nanomatériaux hybrides à base de chitosan, et
- des polymères conducteurs éventuellement modifiés par des fonctions positives.

6. Système de détection électrochimique selon l'une quelconque des revendications précédentes, **caractérisé en ce que** ledit matériau conducteur est formé de carbone, en particulier des nanotubes de carbone, de carbone vitreux, de graphite, de graphène ou d'un métal.

7. Système de détection électrochimique selon l'une quelconque des revendications précédentes, **caractérisé en ce que** ledit matériau conducteur est absent dudit système lorsque ledit nanomatériau porteur de fonctions positives est conducteur.

8. Système de détection électrochimique selon l'une quelconque des revendications précédentes, **caractérisé en ce que** ladite molécule redox est choisie dans le groupe comprenant le ferrocène, la quinone, le bleu de méthylène, les métalloporphyrines, le viologène.

9. Système de détection électrochimique selon l'une quelconque des revendications précédentes, **caractérisé en ce que** les sondes oligonucléotidiques ciblent au moins une molécule d'intérêt choisie indépendamment parmi un ADN, un ARN, une protéine ou une toxine.

10. Support modifié pour la préparation du système de détection selon l'une quelconque des revendications précédentes, comprenant :
- un matériau conducteur,
- un nanomatériau portant des fonctions positives, et
- au moins une molécule redox,
ledit nanomatériau étant situé entre ledit matériau conducteur et ladite molécule redox et étant lié covalemment à ces deux derniers.

11. Kit de préparation d'un système de détection électrochimique selon l'une quelconque des revendications 1 à 9, comprenant:
(i) un support modifié tel que défini selon la revendication 10, et
(ii) au moins un oligonucléotide ciblant une molécule d'intérêt.

12. Méthode de préparation d'un système de détection électrochimique selon l'une quelconque des revendications 1 à 9, comprenant :
(i) la mise en contact d'un support modifié tel que défini selon la revendication 10 avec au moins un oligonucléotide fonctionnalisé ciblant une molécule d'intérêt ;
(ii) la formation des liaisons covalentes entre le susdit support modifié et ledit oligonucléotide fonctionnalisé.

13. Méthode électrochimique de détection d'au moins un type de molécule d'intérêt dans un échantillon, **caractérisée en ce qu'**elle comprend les étapes suivantes :
(i) la mise en contact d'un système de détection tel que défini selon l'une quelconque des revendications 1 à 9 avec un échantillon susceptible de contenir ladite molécule d'intérêt ;
(ii) la mesure électrochimique du signal redox spécifique à ladite molécule d'intérêt.

14. Méthode électrochimique de détection selon la revendication 13, **caractérisée en ce qu'**elle comprend, en plus des étapes (i) et (ii) et après l'étape (ii), le dosage quantitatif de ladite molécule d'intérêt en se rapportant à une courbe de calibration.

15. Méthode électrochimique de détection multiple d'au moins deux types de molécules d'intérêt dans un échantillon, **caractérisée en ce qu'**elle comprend les étapes suivantes :
(i) la mise en contact d'un système de détection tel que défini selon la revendication 3 avec un échantillon susceptible de contenir lesdites molécules d'intérêt ;
(ii) la mesure des signaux redox spécifiques auxdites molécules d'intérêt.

16. Utilisation d'un système de détection électrochimique tel que défini selon l'une quelconque des revendications 1 à 9 ou d'un kit de préparation dudit système selon la revendication 11 pour diagnostiquer *in vitro* ou *in vivo* une maladie, pour identifier la résistance à un médicament et pour suivre la présence d'un médicament dans les fluides biologiques.

17. Utilisation d'un système de détection électrochimique tel que défini selon l'une quelconque des revendications 1 à 9 ou d'un kit de préparation dudit système selon la revendication 11 pour détecter la présence d'un pathogène ou une toxine dans un échantillon agroalimentaire, pharmaceutique ou un effluent.

## Patentansprüche

1. Elektrochemisches Nachweissystem für Zielmoleküle, **dadurch gekennzeichnet, dass** das System umfasst:
- ein leitfähiges Material,
- mindestens ein Nanomaterial mit positiven Funktionen, das kovalent an das leitfähige Material gebunden ist,
- mindestens ein Redoxmolekül und
- mindestens eine einzelsträngige Oligonukleotidsonde, die auf ein Zielmolekül abzielt,
wobei genanntes Redoxmolekül kovalent an das Nanomaterial gebunden ist;
wobei genannte Oligonukleotidsonde kovalent an das genannte Nanomaterial oder an das Redoxmolekül gebunden ist;
wobei die Zahl der positiven Funktionen des genannten Nanomaterials größer ist als die Zahl der Oligonukleotidsonden.

2. Elektrochemisches Nachweissystem nach Anspruch 1, **dadurch gekennzeichnet, dass** genanntes Redoxmolekül sowohl an genanntes Nanomaterial als auch an die Oligonukleotidsonde kovalent gebunden ist.

3. Elektrochemisches Nachweissystem nach Anspruch 2, **dadurch gekennzeichnet, dass** genanntes System umfasst:
- mindestens zwei Arten von Redoxmolekülen, deren Redoxpotentiale unterschiedlich sind, und
- mindestens zwei Arten einzelsträngiger Oligonukleotidsonden, die jeweils auf ein anderes Zielmolekül abzielen,
wobei die gleiche Art von Oligonukleotidsonden mit der gleichen Art von Redoxmolekülen verbunden ist.

4. Elektrochemisches Nachweissystem nach Anspruch 1, **dadurch gekennzeichnet, dass** genannte Oligonukleotidsonde und genanntes Redoxmolekül jeweils kovalent an genanntes Nanomaterial gebunden sind.

5. Elektrochemisches Nachweissystem nach einem der vorhergehenden Ansprüche, **dadurch gekennzeichnet, dass** das genannte positive Funktionen tragende Nanomaterial aus der Gruppe ausgewählt ist, die umfasst:
- ein Dendrimer, vorzugsweise ein Dendrimer vom Poly(amidoamin)-Typ, insbesondere der zweiten, vierten oder sechsten Generation,
- metallische Partikel wie Goldnanopartikel,
- magnetische Nanopartikel wie Eisenoxid,
- hybride Nanomaterialien auf Basis von Chitosan, und
- leitfähige Polymere, die gegebenenfalls durch positive Funktionen modifiziert sind.

6. Elektrochemisches Nachweissystem nach einem der vorhergehenden Ansprüche, **dadurch gekennzeichnet, dass** genanntes leitfähiges Material aus Kohlenstoff, insbesondere Nanoröhren aus Kohlenstoff, Glaskohlenstoff, Graphit, Graphen oder einem Metall gebildet ist.

7. Elektrochemisches Nachweissystem nach einem der vorhergehenden Ansprüche, **dadurch gekennzeichnet, dass** genanntes leitfähiges Material in dem System fehlt, wenn das positive Funktionen tragende Nanomaterial leitfähig ist.

8. Elektrochemisches Nachweissystem nach einem der vorhergehenden Ansprüche, **dadurch gekennzeichnet, dass** genanntes Redoxmolekül aus der Gruppe ausgewählt ist, die Ferrocen, Chinon, Methylenblau, Metalloporphyrine, Viologen umfasst.

9. Elektrochemisches Nachweissystem nach einem der vorhergehenden Ansprüche, **dadurch gekennzeichnet, dass** die Oligonukleotidsonden auf mindestens ein Zielmolekül abzielen, das unabhängig ausgewählt ist aus einer DNA, einer RNA, einem Protein oder einem Toxin.

10. Modifizierter Träger zur Herstellung des Nachweissystems nach einem der vorhergehenden Ansprüche, umfassend:
- ein leitfähiges Material,
- ein Nanomaterial, das positive Funktionen trägt und
- mindestens ein Redoxmolekül,
wobei sich genanntes Nanomaterial zwischen dem genannten leitfähigen Material und dem genannten Redoxmolekül befindet und an die beiden Letzteren kovalent gebunden ist.

11. Kit zur Herstellung eines elektrochemischen Nachweissystems nach einem der Ansprüche 1 bis 9, umfassend:
(i) einen modifizierten Träger wie nach Anspruch 10 definiert, und
(ii) mindestens ein Oligonukleotid, das auf ein Zielmolekül abzielt.

12. Verfahren zur Herstellung eines elektrochemischen Nachweissystems nach einem der Ansprüche 1 bis 9, umfassend:
(i) Inkontaktbringen eines modifizierten Trägers wie nach Anspruch 10 definiert mit mindestens einem funktionalisierten Oligonukleotid, das auf ein Zielmolekül abzielt;
(ii) Bildung kovalenter Bindungen zwischen dem oben genannten modifizierten Träger und dem genannten funktionalisierten Oligonukleotid.

13. Elektrochemisches Verfahren zum Nachweis mindestens einer Zielmolekülart in einer Probe, **dadurch gekennzeichnet, dass** es die folgenden Schritte umfasst:
(i) Inkontaktbringen eines Systems zum Nachweis nach einem der Ansprüche 1 bis 9 mit einer Probe, die genanntes Zielmolekül enthalten kann;
(ii) elektrochemische Messung des für genanntes Zielmolekül spezifischen Redoxsignals.

14. Elektrochemisches Verfahren zum Nachweis nach Anspruch 13, **dadurch gekennzeichnet, dass** es zusätzlich zu den Schritten (i) und (ii) und nach Schritt (ii) die quantitative Messung des genannten Zielmoleküls in Bezug auf eine Eichkurve umfasst.

15. Elektrochemisches Verfahren zum Mehrfachnachweis von mindestens zwei Arten von Zielmolekülen in einer Probe, **dadurch gekennzeichnet, dass** es die folgenden Schritte umfasst:
(i) Inkontaktbringen eines Nachweissystems wie definiert nach Anspruch 3 mit einer Probe, die genannte Zielmoleküle enthalten kann;
(ii) Messung der für genannte Zielmoleküle spezifischen Redoxsignale.

16. Verwendung eines elektrochemischen Nachweissystems wie nach einem der Ansprüche 1 bis 9 definiert oder eines Kits zur Herstellung des genannten Systems nach Anspruch 11 zum Diagnostizieren einer Erkrankung *in vitro* oder *in vivo,* zum Identifizieren einer Resistenz gegen ein Arzneimittel und zum Überwachen des Vorhandenseins eines Arzneimittels in Körperflüssigkeiten.

17. Verwendung eines elektrochemischen Nachweissystems wie nach einem der Ansprüche 1 bis 9 definiert oder eines Kits zur Herstellung des genannten Systems nach Anspruch 11 zum Nachweis des Vorhandenseins eines Pathogens oder eines Toxins in einer Nahrungsmittel-, pharmazeutischen oder Abwasserprobe.

## Claims

1. System for electrochemical detection of molecules of interest, **characterized in that** said system comprises:
- a conductive material,
- at least one nanomaterial bearing positive functions, covalently bound to said conductive material,
- at least one redox molecule, and
- at least one single-stranded oligonucleotide probe targeting a molecule of interest,
said redox molecule being covalently bound to said nanomaterial; said oligonucleotide probe being covalently bound to said nanomaterial or to the redox molecule;
the number of positive functions of said nanomaterial being greater than the number of oligonucleotide probes.

2. Electrochemical detection system according to claim 1, **characterized in that** said redox molecule is covalently bound both to said nanomaterial and to the oligonucleotide probe.

3. Electrochemical detection system according to claim 2, **characterized in that** said system comprises:
- at least two types of redox molecules the oxidation-reduction potentials of which are different, and
- at least two types of single-stranded oligonucleotide probes each targeting a different molecule of interest,
the same type of oligonucleotide probes being bound to the same type of redox molecules.

4. Electrochemical detection system according to claim 1, **characterized in that** said oligonucleotide probe and said redox molecule are respectively covalently bound to said nanomaterial.

5. Electrochemical detection system according to any one of the preceding claims, **characterized in that** said nanomaterial bearing positive functions is selected from the group comprising:
- a dendrimer, preferentially a dendrimer of the poly(amidoamine) type, in particular of second, fourth or sixth generation,
- metal particles such as gold nanoparticles,
- magnetic nanoparticles, such as iron oxide,
- hybrid nanomaterials based on chitosan, and
- conductive polymers, optionally modified by positive functions.

6. Electrochemical detection system according to any one of the preceding claims, **characterized in that** said conductive material is formed from carbon, in particular from carbon nanotubes, glassy carbon, graphite, graphene or a metal.

7. Electrochemical detection system according to any one of the preceding claims, **characterized in that** said conductive material is absent from said system when said nanomaterial bearing positive functions is conductive.

8. Electrochemical detection system according to any one of the preceding claims, **characterized in that** said redox molecule is selected from the group comprising ferrocene, quinone, methylene blue, metalloporphyrins, viologen.

9. Electrochemical detection system according to any one of the preceding claims, **characterized in that** the oligonucleotide probes target at least one molecule of interest selected independently from a DNA molecule, a RNA molecule, a protein or a toxin.

10. Modified support for the preparation of the detection system according to any one of the preceding claims, comprising:
- a conductive material,
- a nanomaterial bearing positive functions, and
- at least one redox molecule,
said nanomaterial being situated between said conductive material and said redox molecule and being covalently bound to the latter two.

11. Kit for the preparation of an electrochemical detection system according to any one of claims 1 to 9, comprising:
(i) a modified support as defined in claim 10, and
(ii) at least one oligonucleotide targeting a molecule of interest.

12. Method for the preparation of an electrochemical detection system according to any one of claims 1 to 9, comprising:
(i) bringing a modified support as defined in claim 10 into contact with at least one functionalized oligonucleotide targeting a molecule of interest;
(ii) forming covalent bonds between the aforementioned modified support and said functionalized oligonucleotide.

13. Electrochemical method for the detection of at least one type of molecule of interest in a sample, **characterized in that** it comprises the following steps:
(i) bringing a detection system as defined in any one of claims 1 to 9 into contact with a sample capable of containing said molecule of interest;
(ii) electrochemical measurement of the redox signal specific to said molecule of interest.

14. Electrochemical detection method according to claim 13, **characterized in that** it comprises, in addition to steps (i) and (ii) and after step (ii), the quantitative analysis of said molecule of interest, with reference to a calibration curve.

15. Electrochemical method for the multiple detection of at least two types of molecules of interest in a sample, **characterized in that** it comprises the following steps:
(i) bringing a detection system as defined in claim 3 into contact with a sample capable of containing said molecules of interest;
(ii) measuring the redox signals specific to said molecules of interest.

16. Use of an electrochemical detection system as defined in any one of claims 1 to 9 or a kit for the preparation of said system according to claim 11 for the *in vitro* or *in vivo* diagnosis of a disease, in order to identify resistance to a medicinal product and in order to monitor the presence of a medicinal product in the biological fluids.

17. Use of an electrochemical detection system as defined in one of claims 1 to 9 or a kit for the preparation of said system according to claim 11 for detecting the presence of a pathogen or a toxin in an agri-food or pharmaceutical sample or an effluent.
